Europäisches Patentamt

European Patent Office

Office européen des brevets

(11) Publication number: 0 046 036
B1

# EUROPEAN PATENT SPECIFICATION

(45) Date of publication of patent specification: **10.10.84**

(21) Application number: **81303506.0**

(22) Date of filing: **31.07.81**

(51) Int. Cl.³: **A 61 K 9/06,** A 61 K 9/10, A 61 K 7/48

(54) Compositions containing povidone-iodine.

(30) Priority: **02.08.80 DE 3029477**
**29.05.81 GB 8116395**

(43) Date of publication of application:
**17.02.82 Bulletin 82/07**

(45) Publication of the grant of the patent:
**10.10.84 Bulletin 84/41**

(84) Designated Contracting States:
**AT BE CH DE FR GB IT LI LU NL SE**

(56) References cited:
DE-A-2 539 462
US-A-3 687 855
US-A-4 125 602

JANISTYN H., Kosmetischen Grundstoffe,
Handbuch der Kosmetika und Riechstoffe, vol.
1, Huthig Verlag, 1978, Heidelberg, DE;

(73) Proprietor: SMITHKLINE DAUELSBERG GmbH
& COMPANY
Postfach 324
D-3400 Gottingen (DE)

(72) Inventor: Gottwald, Eberhard Fritz
Plessestrasse 8
D-3406 Bovenden 1 (DE)
Inventor: Machoczek, Horst Manfred
Allensteiner Weg 28
D-3407 Gleichen-Reinhausen (DE)

(74) Representative: Johnston, Walter Paul et al
P.O. Box 39
Welwyn Garden City Hertfordshire AL7 1EY (GB)

## Description

This invention relates to pharmaceutical compositions containing polyvinylpyrrolidone-iodine complex, to a process for their preparation and their use at topical disinfectants.

Polyvinylpyrrolidone-iodine complex, also referred to as povidone-iodine (see Martindale, the Extra Pharmacopoeia, 27th Ed., p 830) is a well known topical disinfectant. It has antifungal, antibacterial, antiprotazoal and antiviral activity. It has been used for many years (usually as an ointment) for the prevention and treatment of topical infections, including wound infections. A well known polyvinylpyrrolidone-iodine ointment is that sold under the name BETAISODONA or BETADINE (BETAISODONA and BETADINE are Registered Trade Marks).

The disadvantage of known compositions containing polyvinylpyrrolidone-iodine complex is that they are dark brown and, when applied topically, visibly discolour the skin or mucosa. Some patients find the discolouration and the dark colour of the compositions so distasteful that they fail to comply with prescribed treatment.

Although the development of polyvinylpyrrolidone-iodine complex compositions that do not discolour the skin and that do not have a dark brown colour has been long recognised as desirable, no practical solution to this discolouration problem has previously been proposed. The addition of white pigment to known polyvinylpyrrolidone compositions (which have aqueous carriers) has little or no effect on their colour.

According to the present invention there is provided a pharmaceutical composition suitable for topical application comprising particulate polyvinyl pyrrolidone-iodine complex dispersed in, as a continuous phase a substantially anhydrous pharmaceutically acceptable carrier in which the complex is substantially insoluble and from which the complex is released when the composition is applied topically and a light coloured pigment dispersed therein such that the colour of the composition falls within the region described by the following equation:—

$$1.4879R_1^2 + 0.029495Y_1^2 + 5.0665B_1^2 - 0.34806R_1Y_1 - 5.3486R_1B_1 + 0.54292Y_1B_1 = <1$$

where

| | | |
|---|---|---|
| $R_1$=Lovibond Red | 4.3111 |
| $Y_1$=Lovibond Yellow | 6.2167 |
| $B_1$=Lovibond Blue | 1.8833 |

The above equation expresses the range of colours for the compositions of the invention using the Lovibond scale. This scale expresses a colour as a combination of three primary colours red, yellow and blue. The colour is measured on the Lovibond scale using a Lovibond Tintometer. The equation above is derived empirically by measuring the colours of a range of compositions.

The compositions described by this equation vary in colour from white through buff to deep tan.

Compositions where the colour falls within the region described by the equation:—

$$1.9946\ R_2^2 + 0.061339\ Y_2^2 + 6.3034B_2^2 - 0.599R_2Y_2 - 6.9148R_2B_2 + 0.92198Y_2B_2 = <1$$

where

| | | |
|---|---|---|
| $R_2$=Lovibond Red | 3.9000 |
| $Y_2$=Lovibond Yellow | 5.0375 |
| $B_2$=Lovibond Blue | 1.7187 |

vary in color from white through buff to mid-tan.

Compositions where the colour falls within the region described by the equation:—

$$2.0761R_3^2 + 0.1037Y_3^2 + 6.449B_3^2 - 7.0766R_3B_3 + 0.9375Y_3B_3 = <1$$

where

| | | |
|---|---|---|
| $R_3$=Lovibond Red | 3.5071 |
| $Y_3$=Lovibond Yellow | 4.0143 |
| $B_3$=Lovibond Blue | 1.5786 |

vary in colour from white through buff to light tan.

Compositions falling within the region described by the equation:—

$$3.7767R_4^2 + 0.31564Y_4^2 + 8.0868B_4^2 - 1.32686R_4Y_4 - 10.4804R_4B_4 + 1.65Y_4B_4 = <1$$

where

| | | |
|---|---|---|
| $R_4$=Lovibond Red | 4.4571 |
| $Y_4$=Lovibond Yellow | 5.7571 |
| $B_4$=Lovibond Blue | 1.9643 |

vary in colour from buff to light tan.

Preferably the compositions fall within the region described by the equation:—

$$6.8076R_5^2 + 0.62875Y_5^2 + 12.2729B_5^2 - 3.0476R_5Y_5 - 17.5926R_5B_5 + 3.1056Y_5B_5 = <1$$

where

| | | |
|---|---|---|
| $R_5$=Lovibond Red | 3.9750 |
| $Y_5$=Lovibond Yellow | 4.4125 |
| $B_5$=Lovibond Blue | 1.7750 |

These compositions are buff-coloured.

In the above equations Lovibond Red, Lovibond Yellow and Lovibond Blue mean the Red, Yellow and Blue values measured for a composition of the invention on the Lovibond scale.

The advantage of the compositions of this invention is that they are a relatively light colour and discolouration is diminished.

In practice less than 10% by weight of the complex has a particle size greater than 150 $\mu$m. Preferably less than 10% by weight of the complex has a particle size greater than 100 $\mu$m. In particular less than 10% by weight of the complex has a particle size of less than 10 $\mu$m. For example 80% by weight of the particulate complex has a particle size in the range 10—50 $\mu$m.

Preferably 90% by weight of the particulate complex has a particle size in the range 20—80 $\mu$m.

The compositions of the invention contain an effective amount of polyvinylpyrrolidine-iodine complex. Typically, this is from 2 to 20% by weight of the composition. In particular it is 10% by weight.

Preferably the compositions contain a buffer so that their pH is from 3.5 to 5.0. (The pH is determined by shaking a sample of the composition with an equal volume of water and measuring the pH of the water).

Examples of buffers which can be used in the compositions of this invention are sodium acetate, disodium hydrogen phosphate and trisodium phosphate.

"Substantially anhydrous" herein means that the carrier contains not more than 3% by weight of water as measured by the Karl-Fischer method.

Preferably the maximum water content is less than 1% by weight. In particular it can be 0.5% by weight.

By "a carrier which releases complex when applied topically" is meant that on administration the carrier releases the complex to an area to which it has been applied. This can be demonstrated *in vitro* by testing a sample of the composition for antibacterial activity on an agar plate.

By "a carrier in which the polyvinylpyrrolidone-iodine complex is substantially insoluble" is meant that the solubility of the complex in the carrier is less than 0.75% by weight.

Preferably the carrier is one where the solubility of the complex is less than 0.2% by weight.

It is of course understood that the carrier is substantially inert to iodine from the polyvinylpyrrolidone-iodine complex, by which it is meant that the carrier has an iodine value of not more than 2. Preferably the iodine value of the carriers is less than 1.

Examples of substances which can be used as carriers in the compositions of this invention are high boiling liquid paraffins and petroleum jellies, higher fatty alcohols and carboxylic acid esters.

An example of a high boiling liquid paraffin is liquid paraffin B.P. An example of a petroleum jelly is soft white paraffin B.P. Examples of higher fatty alcohols are $C_{10}$—$C_{20}$ fatty alcohols, especially dodecanol (lauryl alcohol), tetradecanol (myristyl alcohol), hexadecanol (cetyl alcohol), octadecanol (stearyl alcohol) and an example of a common mixture of such alcohols is cetostearyl alcohol.

The carboxylic acid ester can be an ester of a fatty acid and a fatty alcohol, or a fatty acid mono-, di-, or triglyceride, or a mixture of such compounds. Where the ester is of a fatty acid and fatty alcohol, preferably it contains a total of from 9 to 25 carbon atoms. Thus the fatty alcohol ester containing the smallest number of carbon atoms is octanyl acetate and the fatty acid ester containing the smallest number of carbon atoms is methyl octanoate. Where the ester is a triglyceride, each fatty acid residue has at least 3 and at most 20 carbon atoms. The di- and tri- glycerides can be simple, that is, all the fatty acids in the glyceride are the same, or complex, that is, each glyceride contains two more different fatty acids.

Preferably the carrier is an emulsifying carrier which in particular can form an emulsion on contact with an aqueous medium (e.g. water or serum) at the interface between the carrier and the medium.

The bioavailability of polyvinylpyrrolidone-iodine complex from a composition for example in ointment, can be demonstrated quantitatively in the following test:

A quantity of a composition (1g) is weighed out accurately into a measured volume (30 ml) of physiological salt (0.9% sodium chloride), and the mixture is shaken for from 0.5 to 5.0 min. The mixture is centrifuged (2 min; 3000 rpm), and an aliquot (20 ml) of the supernatant liquid is withdrawn and titrated with 0.01 N sodium thiosulphate. U.V. spectroscopic studies show that polyvinylpyrrolidone-iodine complex is liberated quantitatively so that the measure of available iodine (U.S.P. XX, NF XV—p 647) in the above tests is a measure of complex liberated from the composition.

The above test shows that an ointment consisting of 10% by weight of polyvinylpyrrolidone-iodine complex and 3% by weight of titanium dioxide dispersed in a triglyceride releases almost 100% of the complex.

Preferably the carrier is one which can liberate at least 80% of the complex and in particular at least 50% of the complex.

The emulsifying carrier can be a substance having a hydrophile-lipophile balance such that it can form an emulsion or a mixture of substances (for example a surfactant and a substance of high lipophilicity) such that the mixture has a hydrophile-lipophile balance such that the mixture can form an emulsion.

Examples of substances which form emulsions are higher fatty alcohols, fatty acid esters and mono-, di- and triglycerides and mixtures thereof.

Examples of substances of high lipophilicity which form emulsions when mixed with a surfactant are petroleum jellies and high boiling liquid paraffins. The surfactant can be selected in accordance with normal procedure for making emulsions. Thus the surfactant will be miscible with substance of high lipophilicity. It will also of course be substantially inert to iodine, that is, the carrier containing the surfactant will have an iodine value of less than 2. The surfactant can be one of those substances listed above as being able to form emulsions for example a mono-, or di-glyceride, a higher fatty alcohol (especially cetyl alcohol, stearyl alcohol and cetostearyl alcohol) or a non-ionic polyethylene-oxy surfactant.

The carrier can also contain minor amounts of other constituents which are standard in topical pharmaceutical formulations, for example softeners, thickeners, suspension stabilizers, stiffeners and emollients. Examples of softeners are $C_{1-4}$ alkyl esters of dodecanoic (lauric) and tetradecanoic (myristic) acid, especially isopropyl myristate. An example of a suspension stabiliser is bentonite. Examples of thickeners are silica gels and silica aerosols. Examples of stiffeners are hexadecanol (cetyl alcohol), octadecanol (stearyl alcohol), cetostearyl alcohol and hard white paraffin. Examples of emollients are hexadecanol and isopropyl myristate.

Preferably the carrier consists essentially of a triglyceride (that is the triglyceride is at least 60% by weight of the carrier) with minor proportions of a surfactant and/or the other constituents referred to above.

Preferably the triglyceride has a melting point of 30—60°C. In particular when the composition is an ointment, the triglyceride can have a melting point of 34—40°C and when the composition is a pessary the triglyceride has a melting point of 34—42°C.

The compositions of this invention also contains a light-coloured pigment which lightens the colour of the composition to the required tint.

For example the pigment can be white, preferably it is an insoluble inorganic pigment especially a white metal oxide or sulphate, for example, titanium dioxide, zirconium dioxide or barium sulphate. Preferably it is titanium dioxide. The advantage of titanium dioxide is that it has a particularly strong whitening effect in the compositions of the invention and can be used in small amounts.

When the pigment is titanium dioxide, preferably the titanium dioxide content is 0.5% at least by weight of composition. In particular the content can be from 1, 2 or 3% by weight. In practice the maximum content is not greater than 30% by weight. In particular the content can be up to 20 or 25% by weight. For example the content can be 2, 5 or 10% by weight.

The compositions of the invention are in a form suitable for topical administration. For example the composition can be in the form of a lotion or an ointment for application to the skin, a spreadable cosmetic stick (for example a lipstick) or a pessary.

In an ointment of this invention the carrier typically consists of a petroleum jelly or triglyceride, an emulsifying agent and a softening agent. In a pessary composition of this invention, typically the carrier consists of a triglyceride, an emulsifying agent and a stiffener. The carrier in a cosmetic stick of this invention typically consists of a triglyceride, an emulsifying agent, a stiffener and a thickener.

The compositions of this invention can be prepared by mixing the particulate polyvinylpyrrolidone-iodine complex, with the carrier and any pigment that is to be included, and optionally forming the composition into a shape.

The particulate complex can be mixed with a pigment and this complex-pigment mixture can be mixed with the carrier; the complex, a pigment and carrier can be mixed simultaneously or pigment can be added to a mixture of complex and carrier.

Where the composition is a pessary or cosmetic stick, it is formed into a shape. This can be done by moulding (that is, molten composition is poured into a mould and allowed to cool).

Polyvinylpyrrolidone-iodine complex of the particle size required can be prepared by milling commercially available material and passing it through screens.

The compositions of the invention can be used to treat topical infections in the same way as known polyvinylpyrrolidone-iodine compositions.

The following Examples illustrate the invention.

Example 1

Polyvinylpyrrolidone-iodine complex (particle size 20—80 $\mu$m; 100 g) was mixed with titanium dioxide (100 g) and added to a triglyceride (800 g; m.p. 37—40°C; max. iodine value 1; $C_{12}$—$C_{18}$ fatty acid).

The mixture was heated to the melting point of the triglyceride with continuous stirring. When the mixture was homogeneous, it was poured into ovula pessary moulds and allowed to cool, to give buff-coloured dosage units.

Example 2

Polyvinylpyrrolidone-iodine complex (particle size 20—80 $\mu$m; 100 g) and titanium dioxide (50 g) were mixed and added to a mixture of low melting triglyceride (600 g; $C_8$—$C_{12}$ fatty acid; max. iodine value 1; m.p. less than 10°C) and cetostearyl alcohol (250 g; m.p. 48—52°C; sold under the trade name Lanette O) and the mixture was heated to 50—60°C with con-

tinuous stirring. Low melting $C_8$—$C_{12}$ triglycerides are sold under the trade names Miglyol 812 and Neutralöl.

When mixing was complete, the mass was allowed to cool and homogenised on a roller frame. The product (an extrudable ointment) was filled into tubes.

### Example 3

Polyvinylpyrrolidone-iodine complex (particle size 20—80 $\mu$m; 100 g) was mixed with titanium dioxide (20 g) and this mixture was added to a pre-heated (50°C) mixture of high-melting triglyceride (440 g; $C_{12}$—$C_{18}$ fatty acid m.p. 35°C; max. iodine value 1;) and liquid triglyceride (440 g; $C_8$—$C_{12}$ fatty acid; m.p. less than 10°C; max. iodine value 1; sold under the trade name Myritrol 318), with continuous stirring. The mass was allowed to cool the resultant ointment was homogenised on a roller frame and filled into tubes.

### Example 4

Polyvinylpyrrolidone-iodine complex (particle size 20—80 $\mu$m; 100 g) was mixed with titanium dioxide (50 g) and added to a hot (55—60°C) mixture of high melting triglyceride (550 g; m.p. 30—42°C; max. iodine value 1; $C_{12}$—$C_{18}$ fatty acids), glyceryl monostearate (50 g; m.p. 56—60°C) and cetyl alcohol (50 g) with continuous stirring. The resultant mixture was warmed (ca 70—80°C) to increase mobility, homogenised, filled into moulds and allowed to cool to produce buff-coloured cosmetic sticks.

### Example 5

Polyvinylpyrrolidone-iodine complex (20—80 $\mu$m; 100 g) was mixed with titanium dioxide (30 g) and added to a preheated (approx 35°C) mixture of plastibase (840 g) and cetyl alcohol (50 g). Plastibase is a mixture of liquid paraffin and a polyethylene (approx M. W. 21000). When uniform, the mixture was allowed to cool, homogenised on a roller frame and the resultant ointment was filled into collapsible tubes.

### Example 6

(a) A mixture of polyvinylpyrrolidone-iodine (20—80 $\mu$m; 100 g) and titanium dioxide (30g) was mixed with a heated (approx 35°C) mixture of plastibase (840 g) and emulsifier (10g; Cremophor (RH40, mixture of castor oil, esters and polyethylene glycol). The product was allowed to cool and was then homogenised on a roller frame and the ointment was filled into collapsible tubes.

(b) Brij 35 [0.5%, a polyoxyethylene fatty alcohol ether], is used as an alternative emulsifier in the composition of Example 6(a).

### Example 7

A mixture of polyvinylpyrrolidone-iodine complex (20—80 $\mu$m; 100 g) and titanium dioxide (30 g) was added to a heated mixture of soft white paraffin B.P. (730 g) and cetostearyl alcohol (140 g) with continuous stirring. The product was allowed to cool and was homogenised on roller frames and the resultant ointment was filled into collapsible tubes.

### Example 8

(a) A mixture of polyvinylpyrrolidone-iodine complex (20—80 $\mu$m; 100 g) and titanium dioxide (30 g) was added with stirring to a heated (approx 35°C) mixture of soft white paraffin B.P. and emulsifier (10 g; Cremophor RH40). The product was allowed to cool and was homogenised on a roller frame and the ointment was filled into collapsible tubes.

(b) Brij 35 [0.5%, a polyoxyethylene fatty alcohol ether], is used as an alternative emulsifier in the composition of Example 8(a).

### Example 9

A mixture of polyvinylpyrrolidone-iodine complex (20—80 $\mu$m; 100 g) titanium dioxide (30 g) and Aerosil (Registered Trade Mark) (3 g) were added with continuous stirring to a heated (ca 60—70°C) mixture of cetostearyl alcohol (300 g) liquid triglyceride (610 g; m.p. less than 10°C; $C_3$—$C_{18}$ fatty acids; iodine value 1) and polyvinylpyrrolidone (20 g). The mixture was homogenised on a roller frame and filled into moulds to produce cosmetic sticks.

### Example 10

(a) A mixture of polyvinylpyrrolidone-iodine complex (20—80 $\mu$m; 100 g), titanium dioxide (30 g) and Aerosil (Registered Trade Mark) (10 g) was added to a heated (approx 45°C) mixture of cetostearyl alcohol (300 g), high-melting triglyceride (110 g; $C_8$—$C_{18}$ fatty acid; max. iodine value 1; m.p. 30—42°C), medium-melting triglyceride (11o g; $C_8$—$C_{18}$ fatty acid; max. iodine value 1 m.p. 35—40°C) and low-melting triglyceride (200 g; $C_8$—$C_{12}$ fatty acid; max. iodine value 1; m.p. less than 10°C; sold under the trade name Miglyol 812) with continuous stirring. To this mixture was then added 1% (10 g) of isopropyl myristate with stirring. The mixture was heated to a temperature of between 40 and 45°C and poured into ovula pessary moulds.

(b) in the formulation of Example 10(a) Miglyol 812 is replaced by Myritrol 318.

The compositions of Examples 1 to 10 can be buffered to pH 3.5—4.5 by adding from 0.3 to 0.4% sodium acetate by weight of the composition.

### Example 11

High-melting triglyceride (790 g; $C_{12}$—$C_{18}$ fatty acid; max. iodine value 1; m.p. 30—42°C) and low-melting triglyceride (50 g; ($C_8$—$C_{12}$ fatty acid, max. iodine value 1; m.p. less than 10°C) were heated together with stirring until molten (ca 40°C). To this molten mixture was added with stirring silica [5.0 g; Aerosil (Registered Trade Mark)], titanium dioxide (50 g), polyvinylpyrrolidone-iodine complex (20—80 $\mu$m; 100 g) and sodium acetate (4 g). The mixture was stirred and heated to keep it fluid until homogeneous and poured into ovula pessary moulds and allowed to cool.

Example 12

Polyvinylpyrrolidone-iodine complex (particle size 20 to 80 $\mu$m; 100 g) was mixed with titanium dioxide (50 g) and added to a hot (55—60°C) mixture of low-melting triglyceride (185 g; $C_8$—$C_{12}$ fatty acid; m.p. less than 10°C; max. iodine value 1) and an organic modified montmorillonite (sold under the trade name Bentone). A commercially available mixture of this type is sold under the Trade name Miglyol gel. To this mixture was then added medium-melting triglyceride (250 g; $C_8$—$C_{18}$ fatty acid; m.p. 35—40°C; max. iodine value 1) high melting triglyceride (300 g; $C_{12}$—$C_{18}$ fatty acid m.p. 30—42°C max. iodine value 1) with continuous stirring.

The compositions of the above Examples are buff-coloured.

**Claims for the Contracting States; BE, CH, DE, FR, GB, IT, LI, LU, NL, SE**

1. A pharmaceutical composition suitable for topical application comprising particulate polyvinyl pyrrolidone-iodine complex dispersed in, as a continuous phase, a substanitially anhydrous pharmaceutically acceptable carrier in which the complex is substantially insoluble and from which the complex is released when the composition is applied topically and a light coloured pigment dispersed therein such that the colour of the composition falls within the region described by the following equation:—

$$1.4879R_1^2+0.029495Y_1^2+5.0665B_1^2-$$
$$0.34806R_1Y_1-5.3486R_1B_1+0.54292Y_1B_1=<1$$

where

| | |
|---|---|
| $R_1$=Lovibond Red | 4.3111 |
| $Y_1$=Lovibond Yellow | 6.2167 |
| $B_1$=Lovibond Blue | 1.8833 |

2. A composition as claimed in claim 1, where the colour falls within the region described by the equation:—

$$6.8076R_5^2+0.62875Y_5^2+12.2729B_5^2-$$
$$3.0476R_5Y_5-17.5926R_5B_5+3.1056Y_5B_5=<1$$

where

| | |
|---|---|
| $R_5$=Lovibond Red | 3.9750 |
| $Y_5$=Lovibond Yellow | 4.4125 |
| $B_5$=Lovibond Blue | 1.7750 |

3. A composition as claimed in claim 1 or 2, where 90% by weight of the complex has a particle size in the range 20 to 80 $\mu$m.

4. A composition as claimed in any one of claims 1 to 3, where the carrier contains less than 3% by weight of water.

5. A composition as claimed in claim 4, where the carrier contains less than 1% by weight of water.

6. A composition as claimed in any one of claims 1 to 5, where solubility of the carrier for the complex is not more than 0.75% by weight.

7. A composition as claimed in claim 6, where solubility of the carrier for the complex is not more than 0.2% by weight.

8. A composition as claimed in any one of claims 1 to 7, where the carrier is an emulsifying carrier.

9. A composition as claimed in claim 8, where the carrier consists essentially of a fatty acid triglyceride.

10. A composition as claimed in claim 9, where the triglyceride is a $C_8$ to $C_{20}$ fatty acid triglyceride.

11. A composition as claimed in claim 10, where the triglyceride has a melting point within the range 30—60°C.

12. A composition as claimed in any one of claims 1 to 11, which further comprises a white pigment dispersed in the carrier.

13. A composition as claimed in any one of claims 1 to 12, in the form of an ointment, a spreadable cosmetic stick or a pessary.

14. A composition as claimed in claim 13 in the form of an ointment, where the carrier is a fatty acid triglyceride and the triglyceride has a melting point within the range 34—40°C.

15. A process for preparing a composition as claimed in any one of Claims 1 to 14, which comprises mixing particulate polyvinylpyrrolidone-iodine complex with a carrier and any pigment that is to be included and optionally forming the composition into a shape.

**Claims for the Contracting State; AT**

1. A process for preparing a pharmaceutical composition suitable for topical application comprising particulate polyvinyl pyrrolidone-iodine complex dispersed in, as a continuous phase, a substantially anhydrous pharmaceutically acceptable carrier in which the complex is substantially insoluble; and from which the complex is released when the composition is applied topically and a light coloured pigment dispersed therein such that the colour of the composition falls within the region described by the following equation:—

$$1.4879R_1^2+0.029495Y_1^2+5.0665B_1^2-$$
$$0.34806R_1Y_1-5.3486R_1B_1+0.54292Y_1B_1=<1$$

where

R$_1$=Lovibond Red    4.3111

Y$_1$=Lovibond Yellow    6.2167

B$_1$=Lovibond Blue    1.8833

which comprises mixing particulate polyvinylpyrrolidine-iodine complex with the carrier and any pigment that is to be included and optionally forming the composition into a shape.

2. A process as claimed in claim 1, where the colour falls within the region described by the equation:—

$$6.8076R_5^2+0.62875Y_5^2+12.2729B_5^2-3.0476R_5Y_5-17.5926R_5B_5+3.1056Y_5B_5=<1$$

where

R$_5$=Lovibond Red    3.9750

Y$_5$=Lovibond Yellow    4.4125

B$_5$=Lovibond Blue    1.7750

3. A process as claimed in claim 1 or 2 where 90% by weight of the complex has a particle size in the range 20 to 80 $\mu$m.

4. A process as claimed in any one of claims 1 to 3, where the carrier contains less than 3% by weight of water.

5. A process as claimed in claim 4, where the carrier contains less than 1% by weight of water.

6. A process as claimed in any one of claims 1 to 5, where solubility of the carrier for complex is not more than 0.75% by weight.

7. A process as claimed in claim 6, where solubility of the carrier for the complex is not more than 0.2% by weight.

8. A process as claimed in any one of claims 1 to 7, where the carrier is an emulsifying carrier.

9. A process as claimed in claim 8, where the carrier consists essentially of a fatty acid triglyceride.

10. A process as claimed in claim 9, where the triglyceride is a C$_8$ to C$_{20}$ fatty acid triglyceride.

## Patentansprüche Für die Vertragsstaaten: BE, CH, DE, FR, GB, IT, LI, LU, NL, SE

1. Eine zur topischen Anwendung geeignete pharmazeutische Zusammensetzung, enthaltend teilchenförmigen Polyvinylpyrrolidon-Jod-Komplex, der in—als kontinuierlicher Phaseeinem im wesentlichen wasserfreiem, pharmazeutisch verträglichen Träger, in dem der Komplex im wesentlichen unlöslich ist und aus dem der Komplex bei topischer Anwendung der Zusammensetzung freigesetzt wird, dispergiert ist, und ein darin dispergiertes, hell gefärbtes Pigment, so dass die Farbe der Zusammensetzung in den durch folgende Gleichung beschriebenen Bereich fällt:

$$1,4879R_1^2+0,029495Y_1^2+5,0665B_1^2-0.34806R_1Y_1-5,3486R_1B_1+0,54292Y_1B_1=<1$$

wobei

R$_1$=Lovibond-Rot    4,3111

Y$_1$=Lovibond-Gelb    5,2167

B$_1$=Lovibond-Blau    1,8833

2. Zusammensetzung nach Anspruch 1, wobei die Farbe in den durch folgende Gleichung definierten Bereich fällt:

$$6,8076R_5^2+0,62875Y_5^2+12,2729B_5^2-3,0476R_5Y_5-17,5926R_5B_5+3,1056Y_5B_5=<1$$

wobei

R$_5$=Lovibond-Rot    3,9750

Y$_5$=Lovibond-Gelb    4,4125

B$_5$=Lovibond-Blau    1,7750

3. Zusammensetzung nach Anspruch 1 oder 2, wobei 90 Gewichtsprozent des Komplexes eine Teilchengrösse im Bereich von 20 bis 80 $\mu$m aufweist.

4. Zusammensetzung nach einem der Ansprüche 1 bis 3, wobei der Träger weniger als 3 Gewichtsprozent Wasser enthält.

5. Zusammensetzung nach Anspruch 4, wobei der Träger weniger als 1 Gewichtsprozent Wasser enthält.

6. Zusammensetzung nach einem der Ansprüche 1 bis 5, wobei die Löslichkeit des Trägers für den Komplex nicht mehr als 0,75 Gewichtsprozent beträgt.

7. Zusammensetzung nach Anspruch 6, wobei die Löslichkeit des Trägers für den Komplex nicht mehr als 0,2 Gewichtsprozent beträgt.

8. Zusammensetzung nach einem der Ansprüche 1 bis 7, wobei der Träger ein emulgierender Träger ist.

9. Zusammensetzung nach Anspruch 8, wobei der Träger im wesentlichen aus einem Fettsäureetriglyceride besteht.

10. Zusammensetzung nach Anspruch 9, wobei es sich beim Triglycerid um ein C$_8$ bis C$_{20}$-Fettsäuretriglycerid handelt.

11. Zusammensetzung nach Anspruch 9, wobei es sich beim Triglycerid un ein C$_8$ bis C$_{20}$-Fettsäuretriglycerid handelt.

11. Zusammensetzung nach Anspruch 10, wobei das Triglycerid einen Schmelzpunkt im Bereich von 30 bis 60°C aufweist.

12. Zusammensetzung nach einem der Ansprüche 1 bis 11, die zusätzlich ein im Träger dispergiertes weisses Pigment enthält.

13. Zusammensetzung nach einem der Ansprüche 1 bis 12 in Form einer Salbe, eines verstreichbaren kosmetischen Stifts oder eines Pessars.

14. Zusammensetzung nach Anspruch 13 in Form einer Salbe, wobei es sich beim Träger um ein Fettsäuretriglycerid handelt und das Triglycerid einen Schmelzpunkt im Bereich von 34 bis 40°C aufweist.

15. Verfahren zur Herstellung einer Zusammensetzung nach einem der Ansprüche 1 bis 14, bei dem man teilchenförmigen Polyvinylpyrrolidon-Jod-Komplex mit dem Träger und einem zuzesetzenden Pigment vermischt und die Zusammensetzung gegebenenfalls in eine gewünschte Form bringt.

## Patentansprüche für den Vertragsstaat: AT

1. Verfahren zur Herstellung einer zur topischen Anwendung geeigneten pharmazeutischen Zusammensetzung, enthaltend teilchenförmigen Polyvinylpyrrolidon-Jod-Komplex, der in—als kontinuierlicher Phaseeinem im wesentlichen wasserfreiem, pharmazeutisch verträglichen Träger, in dem der Komplex im wesentlichen unlöslich ist und aus dem der Komplex bei topischer Anwendung der Zusammensetzung freigesetzt wird, dispergiert ist, und ein darin dispergiertes, hell gefärbtes Pigment, so dass die Farbe der Zusammensetzung in den durch folgende Gleichung beschriebenen Bereich fällt:

$$1,4879R_1^2+0,029495Y_1^2+5,0665B_1^2-$$
$$0,34806R_1Y_1-5,3486R_1B_1+0,54292Y_1B_1=<1$$

wobei

$R_1$=Lovibond-Rot        4,311

$Y_1$=Lovibond-Gelb      6,2167

$B_1$=Lovibond-Blau      1,8833

bei dem man teilchenförmigen Polyvinylpyrrolidon-Jod-Komplex mit dem Träger und einem zuzusetzenden Pigment vermischt und die Zusammensetzung gegebenenfalls in eine gewünschte Form bringt.

2. Verfahren nach Anspruch 1, dadurch gekennzeichnet, dass die Farbe in den durch die folgende Gleichung beschriebenen Bereich fällt:

$$6,8076R_5^2+0,62875Y_5^2+12,2729B_5^2-$$
$$3,0476R_5Y_5-17,5926R_5B_5+3,1056Y_5B_5=<1$$

wobei

$R_5$=Lovibond-Rot        3,9750

$Y_5$=Lovibond-Gelb      4,4125

$B_5$=Lovibond-Blau      1,7750

3. Verfahren nach Anspruch 1 oder 2, wobei 90 Gewichtsprozent des Komplexes eine Teilchengrösse im Berich von 20 bis 80 $\mu$m aufweisen.

4. Verfahren nach einem der Ansprüche 1 bis 3, wobei der Träger weniger als 3 Gewichtsprozent Wasser enthält.

5. Verfahren nach Anspruch 4, wobei der Träger weniger als 1 Gewichtsprozent Wasser enthält.

6. Verfahren nach einem der Ansprüche 1 bis 5, wobei die Löslichkeit des Trägers für den Komplex nicht mehr als 0,75 Gewichtsprozent beträgt.

7. Verfahren nach Anspruch 6, wobei die Löslichkeit des Trägers für den Komplex nicht mehr als 0,2 Gewichtsprozent beträgt.

8. Verfahren nach einem der Ansprüche 1 bis 7, wobei es sich bei dem Träger um einen emulgierenden Träger handelt.

9. Verfahren nach Anspruch 8, wobei der Träger im wesentlichen aus einem Fettsäuretriglycerid besteht.

10. Verfahren nach Anspruch 9, wobei es sich beim Triglycerid um ein $C_8$ bis $C_{20}$-Fettsäuretriglycerid handelt.

## Revendications pour les etats Contractants: BE, CH, DE, FR, GB, IT, LI, LU, NL, SE

1. Une composition pharmaceutique convenable pour une application topique comprenant un complexe particulaire polyvinylpyrrolidone-iode dispersé en une phase continue dans un support substantiellement anhydre, pharmaceutiquement acceptable, dans lequel le complexe est substantiellement insoluble et à partir duquel le complexe est libéré lorsque la composition est appliquée topiquement, et, un pigment de couleur claire dans ce complexe en sorte que la couleur de cette composition se classe dans la gamme décrite par l'équation suivante:—

$$1,4879R_1^2+0,029495Y_1^2+5,0665B_1^2-$$
$$0,34806R_1Y_1-5,3486R_1B_1+0,54292Y_1B_1=<1$$

dans laquelle

$R_1$=Rouge Lovibond      4,3111

$Y_1$=Jaune Lovibond      6,2167

$B_1$=Bleu Lovibond      1,8833

2. Une composition, comme indiqué dans la revendication 1, dans laquelle la teinte se trouve dans la plage décrite par l'équation:

$$6,8076R_5^2+0,62875Y_5^2+12,2729B_5^2-$$
$$3,0476R_5Y_5-17,5926R_5B_5+3,1056Y_5B_5=<1$$

dans laquelle

$R_5$=Rouge Lovibond      3,9750

$Y_5$=Jaune Lovibond      4,4125

$B_5$=Bleu Lovibond      1,7750

3. Une composition comme indiqué dans la revendication 1 ou 2 dans laquelle 90% par poids du complexe a une dimension de particule dans la plage de 20 à 80 $\mu$m.

4. Une composition comme indiqué dans n'importe laquelle des revendications 1 à 3 dans laquelle le support contient moins de 3% par poids d'eau.

5. Une composition comme indiqué à la revendication 4, dans laquelle le support contient moins de 1% par poids d'eau.

6. Une composition comme indiqué dans n'importe laquelle des revendications de 1 à 5, dans laquelle la solubilité du support pour la, complexe n'est pas supérieure à 0,75% par poids.

7. Une composition comme indiqué dans la revendication 6, dans laquelle la solubilité du support pour le complexe n'est pas supérieure à 0,2% par poids.

8. Une composition comme indiqué dans n'importe laquelle des revendications de 1 à 7, dans laquelle le support est un support émulsifiant.

9. Une composition comme indiqué dans la revendication 8, dans laquelle le support consiste essentiellement en une triglycéride d'acide gras.

10. Une composition comme indiqué dans la revendication 9, dans laquelle la triglycéride est une triglycéride d'acide gras $C_8$ à $C_{20}$.

11. Une composition comme indiqué dans la revendication 10, dans laquelle la triglycéride a un point de fonte entre 30 et 50°C.

12. Une composition comme indiqué dans n'importe laquelle des revendications de 1 à 11, qui comprend en outre un pigment blanc dispersé dans le support.

13. Une composition comme indiqué dans n'importe laquelle des revendications de 1 à 12, sous forme de pommade, de batonnet cosmétique à étendre ou d'ovule vaginal.

14. Une composition comme indiqué dans la revendication 13, sous forme de pommade, dans laquelle le support est une triglycéride d'acide gras et la triglycéride a un point de fonte dans la plage de 34—40°C.

15. Une procédé pour préparer la composition comme indiqué dans n'importe laquelle des revendications de 1 à 14, que consiste à mélanger un complexe particulaire de polyvinylpyrrolidone iode avec le support et un quelconque pigment qui doit être inclus et à donner facultativement une forme à la composition.